# EUROPEAN PATENT APPLICATION

(11) **EP 2 012 121 A1**
(43) Date of publication of application: **07.01.2009**
(21) Application number: 08011804.5
(22) Date of filing: 30.06.2008
(51) Int. Cl.: G01N 33/50

(54) **Enzyme electrode and enzyme sensor**

(30) Priority: 04.07.2007 JP 2007176200
(71) Applicant: Funai Electric Advanced Applied Technology Research Institute Inc., Tsukuba-shi Ibaraki 305-0047 (JP); Funai Electric Co., Ltd., Osaka 574-0013 (JP)
(72) Inventor: Shimomura, Takeshi, Tsukuba-shi Ibaraki 305-0047 (JP); Sumiya, Touru, Tsukuba-shi Ibaraki 305-0047 (JP); Masuda, Yuichiro, Tsukuba-shi Ibaraki 305-0047 (JP); Ono, Masatoshi, Tsukuba-shi Ibaraki 305-0047 (JP)
(74) Representative: Grünecker, Kinkeldey, Stockmair & Schwanhäusser Anwaltssozietät

(57) **Abstract**

Disclosed is an enzyme electrode comprising: an electrode; a carbon nanotube layer including a plurality of carbon nanotubes extending directly from the electrode and/or a metallic catalyst immobilized on the electrode; and an enzyme immobilized in the carbon nanotube layer by being sandwiched between the carbon nanotubes.

## Description

### BACKGROUND OF THE INVENTION

### 1. Field of the Invention

The present invention relates to an enzyme electrode and an enzyme sensor using the enzyme electrode.

### 2. Description of Related Art

Conventionally, the research and development of biosensors to detect specific trace substances have been actively performed from the point of view of the application of the biosensors to environmental problems and medical fields.

In particular, an enzyme sensor detects a target substance electrochemically by using, for example, an electrode immobilizing enzymes on the surface thereof. Because the enzymes react with the target substance specifically, the enzyme sensor has a characteristic capable of detecting a target substance in a mixture selectively at comparatively high sensitivity. As the enzyme sensors, for example, a glucose sensor for diabetes testing, a uric acid sensor for gout testing, a urea sensor for renal function testing, and the like, have been put to practical use in the medical field so far.

From the point of view of the realization of a safe, secure, and comfortable society, for example, techniques to detect dwelling environment pollutants, such as formaldehyde and toluene, explosives, such as trinitrotoluene (TNT) powder, narcotics, such as cocaine and heroin, and the like, at high speeds and high sensitivity have been required in recent years. Because these materials exist in a gaseous phase or in a liquid phase at extremely low concentrations, detection sensitivity in a sub ppb level is requested in order to detect the materials, and the enzyme sensor is desired to further increase the speed and the sensitivity thereof.

The enzymes are polymeric proteins, and exhibit activity on the basis of their steric structures. Consequently, the enzymes have a defect of being easily deactivated owing to various external factors. Accordingly, a method of holding the enzymes on appropriate carriers to stabilize the enzymes through the interactions of the carriers and the enzymes (enzyme immobilization method) has been conventionally developed in order to remove the instability (see, for example, introduction to Enzyme Engineering, Corona Publishing Co., Ltd., pp. 16-100 (1995)).

An enzyme sensor is configured to measure an output current value electrochemically by the use of an enzyme electrode immobilizing enzymes on the surface of the electrode physically or chemically with the enzyme immobilization method. An enzyme sensor having high sensitivity can be obtained by increasing the amount of the enzymes immobilized on the enzyme electrode. However, the amount of the enzymes immobilized on the enzyme electrode depends on the effective surface area of the electrode greatly.

Now, because a carbon nanotube shows a semiconductor property, attempts to use the carbon nanotube as an electronic device have been conventionally performed. Because the carbon nanotube is chemically stable and has a very high electric conductivity, the carbon nanotube is fitted to be used as an electron device. Moreover, because the diameter of the carbon nanotube is within a range from about 1 nm to about 20 nm, it is convenient to use the carbon nanotube also as a device of a minute circuit and an electrode.

Moreover, as an electrochemical property of the carbon nanotube, it can be mentioned that the carbon nanotube has catalytic activity higher than that of the other electrode materials. Consequently, when the carbon nanotube is used as an electrode, then an oxidation current and a reduction current become larger at the same electric potential in comparison with those in the case of using the other electrode materials. Consequently, the use of the carbon nanotube leads to the improvement of the detection sensitivity of the electrode (see, for example, Nature, 354, 56 (1991)).

Moreover, as another characteristic of the carbon nanotube, it can be cited that the carbon nanotube has a high aspect ratio. A carbon nanotube has a length of about several µm as against a diameter thereof of several nm, and consequently has the aspect ratio of a thousandfold or more. Therefore, the carbon nanotube can increase the specific surface area thereof. Consequently, the use of the carbon nanotubes as enzyme immobilizing carriers enables the expectation of immobilizing the enzymes at a higher concentration by a larger amount of adsorption in comparison with those of the immobilizing of the enzymes onto a flat surface. Moreover, the use of the carbon nanotube as an electrode leads to the improvement of the sensitivity and the response speed of the electrode because the carbon nanotube reacts on the whole surface thereof (see, for example, Science, 287, 622 (2000)).

Accordingly, an enzyme sensor using carbon nanotubes was proposed.

To put it concretely, for example, an enzyme sensor using an enzyme electrode made by mixing carbon nanotubes and enzymes into a mineral oil and by applying the mixture onto the electrode was proposed (see, for example, Electroanalysis, 14, 1609 (2002)).

Moreover, an enzyme sensor made by growing carbon nanotubes in a direction perpendicular to a minute metallic catalyst array on a substrate, and by immobilizing enzymes on the ends of the carbon nanotubes was also proposed (see, for example, Japanese Patent Application Laid-Open Publication No. 2005-1105).

However, because the carbon nanotubes are applied on the electrode in the enzyme sensor described in Electroanalysis, 14, 1609 (2002), a Schottky barrier is formed between the carbon nanotubes and the electrode, and a large resistance value is measured. In such a state, the characteristics of the carbon nanotubes cannot be utilized, and sufficient detection sensitivity and a response speed cannot be obtained.

Moreover, because the enzymes are immobilized only on the ends of the carbon nanotubes, that is, only on the surface of a carbon nanotube layer, in the enzyme sensor described in Japanese Patent Application Laid-Open Publication No. 2005-1105, the characteristics of the carbon nanotubes cannot be utilized, and it is impossible to attain the high density immobilizing of the enzymes. Furthermore, because there is nothing to keep the steric structures of the enzymes in the enzyme sensor described in Japanese Patent Application Laid-Open Publication No. 2005-1105, the steric structures of the enzymes change according to the changes of external environments, and the activity of the enzymes is lost. Then, the enzyme sensor has a problem of lacking stability and having a shorter operating life.

### SUMMARY OF THE INVENTION

It is an object of the present invention to provide an enzyme electrode capable of detecting a target substance at high sensitivity and at a high speed, having an excellent stability and a longer operating life, and an enzyme sensor using the enzyme electrode.

According to a first aspect of the present invention, there is provided an enzyme electrode comprising:
an electrode;
a carbon nanotube layer including a plurality of carbon nanotubes extending directly from the electrode and/or a metallic catalyst immobilized on the electrode; and
an enzyme immobilized in the carbon nanotube layer by being sandwiched between the carbon nanotubes.

According to a second aspect of the present invention, there is provided an enzyme electrode comprising:
an electrode;
a carbon nanotube layer including a plurality of carbon nanotubes extending directly from the electrode and/or a metallic catalyst immobilized on the electrode;
an enzyme immobilized in the carbon nanotube layer by being sandwiched between the carbon nanotubes;
an outflow preventing section to prevent the enzyme immobilized in the carbon nanotube layer from outflowing;
an electron carrier to accelerate delivery of electrons between the enzyme and the electrode or the carbon nanotubes, and/or a coenzyme to catalyze expression of activity of the enzyme, the electron carrier and/or the coenzyme being introduced in the carbon nanotube layer; and
a hydrophobic insulating section provided around the electrode.

### BRIEF DESCRIPTION OF THE DRAWINGS

The above and other objects, advantages and features of the present invention will become more fully understood from the detailed description given hereinbelow and the appended drawings which are given by way of illustration only, and thus are not intended as a definition of the limits of the present invention, and wherein:
FIG. 1 is a view showing the principal part of an enzyme electrode of the embodiment of the present invention schematically;
FIG. 2A is a plan view of an enzyme sensor of the embodiment of the present invention;
FIG. 2B is a sectional side view of the enzyme sensor of the embodiment of the present invention;
FIG. 3A is a view for illustrating a part of a manufacture method of the enzyme sensor of the embodiment of the present invention;
FIG. 3B is a view for illustrating another part of the manufacture method of the enzyme sensor of the embodiment of the present invention;
FIG. 3C is a view for illustrating a further part of the manufacture method of the enzyme sensor of the embodiment of the present invention;
FIG. 3D is a view for illustrating a still further part of the manufacture method of the enzyme sensor of the embodiment of the present invention;
FIG. 3E is a view for illustrating a still further part of the manufacture method of the enzyme sensor of the embodiment of the present invention;
FIG. 4A is a sectional side view of an enzyme electrode of the embodiment of the present invention in a case of including an anodized film;
FIG. 4B is another sectional side view of the enzyme electrode of the embodiment of the present invention in the case of including the anodized film;
FIG. 5 is a diagram for illustrating the principle of measuring the concentration of a target substance in a sample by an electrochemical measurement method with the enzyme sensor using the enzyme electrode of the embodiment of the present invention;
FIG. 6 is a schematic view showing a measuring device to evaluate an enzyme sensor of a first example;
FIG. 7 is a diagram showing the results (response currents to formaldehyde concentration) obtained by measurement using the enzyme sensor of the first example;
FIG. 8 is a diagram showing the results (changes of the response currents caused by the introduction of formaldehyde) obtained by measurement using the enzyme sensor of the first example;
FIG. 9 is a diagram showing the results (relative responses to time) obtained by measurement using the enzyme sensor of the first example; and
FIG. 10 is a sectional side view of the enzyme sensor of the embodiment of the present invention in the case of including a gas transmitting film.

### DETAILED DESCRIPTION OF THE PREFERRED EMBODIMENT

In the following, the preferred embodiment of an enzyme electrode according to the present invention and an enzyme sensor using the enzyme electrode will be described in detail with reference to the attached drawings. Incidentally, the scope of the invention is not limited to the shown examples.

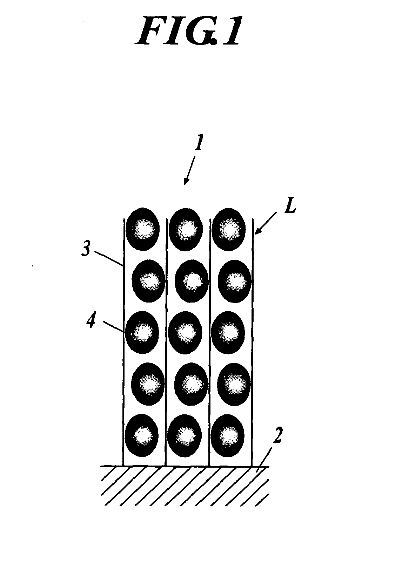

An enzyme electrode 1 according to the embodiment of the present invention is composed of, for example, an electrode 2; a carbon nanotube layer L which includes a plurality of carbon nanotubes 3 which is extending from the electrode 2 and/or a metallic catalyst immobilized on the electrode 2 directly; and enzymes 4 immobilized in the carbon nanotube layer L by being put between the carbon nanotubes 3, as shown in FIG. 1.

The enzymes 4 are, for example, oxidation-reduction enzymes.

However, the enzymes 4 are not limited to the oxidation-reduction enzymes, but are arbitrary as long as they are enzymes (enzyme proteins), and they may be, for example, hydrolytic enzymes, transfer enzymes, and isomerizing enzymes.

Moreover, the enzymes 4 may be, for example, innate enzyme molecules or the fragments of enzymes including active sites. The enzyme molecules or the fragments of the enzymes including the active sites may be, for example, the ones extracted from animals and plants, the ones extracted from microorganisms, the cut ones of them at request, or the ones synthesized by gene engineering or chemical engineering.

To put it concretely, as the oxidation-reduction enzymes, for example, the following enzymes can be used:
glucose oxidase, lactate oxidase, cholesterol oxidase, alcohol oxidase, formaldehyde oxidase, sorbitol oxidase, fructose oxidase, sarcosine oxidase, fructosyl amine oxidase, pyruvic acid oxidase, xanthine oxidase, ascorbic acid oxidase, sarcosine oxidase, choline oxidase, amine oxidase, glucose dehydrogenase, lactate dehydrogenase, cholesterol dehydrogenase, alcohol dehydrogenase, formaldehyde dehydrogenase, sorbitol dehydrogenase, fructose dehydrogenase, hydroxybutyric acid dehydrogenase, glycerol dehydrogenase, glutamate dehydrogenase, pyruvic acid dehydrogenase, malic acid dehydrogenase, glutamic acid dehydrogenase, catalase, peroxidase, and uricase. In addition, cholesterol esterase, creatininase, creatinase, DNA polymerase, mutants of these enzymes, and the like, can be used.

As the hydrolytic enzymes, for example, protease, lipase, amylase, invertase, maltase, β-galactosidase, lysozyme, urease, esterase, a nuclease group, and a phosphatase group can be used.

As the transfer enzymes, for example, various acyltransferases, a kinase group, and an aminotransferase group can be used.

As the isomerizing enzymes, for example, a racemase group, phosphoglycerate phosphomutase, and glucose 6 phosphate isomerase can be used.

The enzymes 4 immobilized in the carbon nanotube layer L may be enzymes of one kind or enzymes of two or more kinds.

To put it concretely, the enzymes 4 immobilized in the carbon nanotube layer L may be, for example, a kind of enzymes, two or more kinds of enzymes having almost the mutually same molecular weights and/or sizes (diameters), or two or more kinds of enzymes having mutually different molecular weights and/or sizes. Moreover, when two or more kinds of enzymes 4 are immobilized in the carbon nanotube layer L, then the enzymes 4 may be, for example, the two or more kinds of enzymes that act on the same kinds of target substances (substrates), the two or more kinds of enzymes that act on different kinds of target substances, or the two or more kinds of enzymes that act on the same and/or different kinds of target substances.

Moreover, when the two or more kinds of enzymes 4 are immobilized in the carbon nanotube layer L, then the two or more kinds of the enzymes may be put between mutually different carbon nanotubes 3 or may be put between mutually the same carbon nanotubes 3 in the carbon nanotube layer L.

In particular, when the two or more kinds of the enzymes 4 are immobilized in the carbon nanotube layer L and the two or more kinds of the enzymes 4 act on mutually different kinds of target substances, then an enzyme sensor 100 can detect the different kinds of target substances (two or more kinds of target substances) at the same time.

The enzyme sensor 100 according to the embodiment of the present invention is a sensor using, for example, the enzyme electrode 1 to detect a target substance by an electrochemical measurement method.

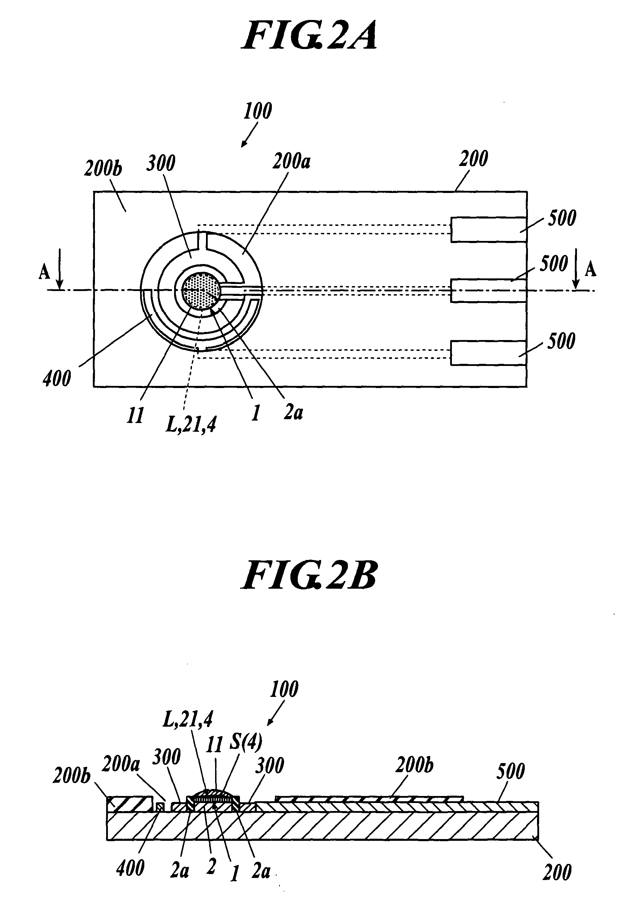

To put it concretely, the enzyme sensor 100 is composed of, for example, a substrate 200, an analysis section 200a provided on the top surface of the substrate 200, which analysis section 200a has an opening portion on the top surface thereof, a hydrophobic insulation film 200b provided around the analysis section 200a on the top surface of the substrate 200, a working electrode (enzyme electrode 1), a counter electrode 300, a reference electrode 400, these three electrodes being disposed in the analysis section 200a on the top surface of the substrate 200, and pads 500 connected to the working electrode (enzyme electrode 1), the counter electrode 300, and the reference electrode 400, respectively, with wiring, as shown in FIGS. 2A and 2B.

### <Manufacturing Method of Enzyme Sensor>

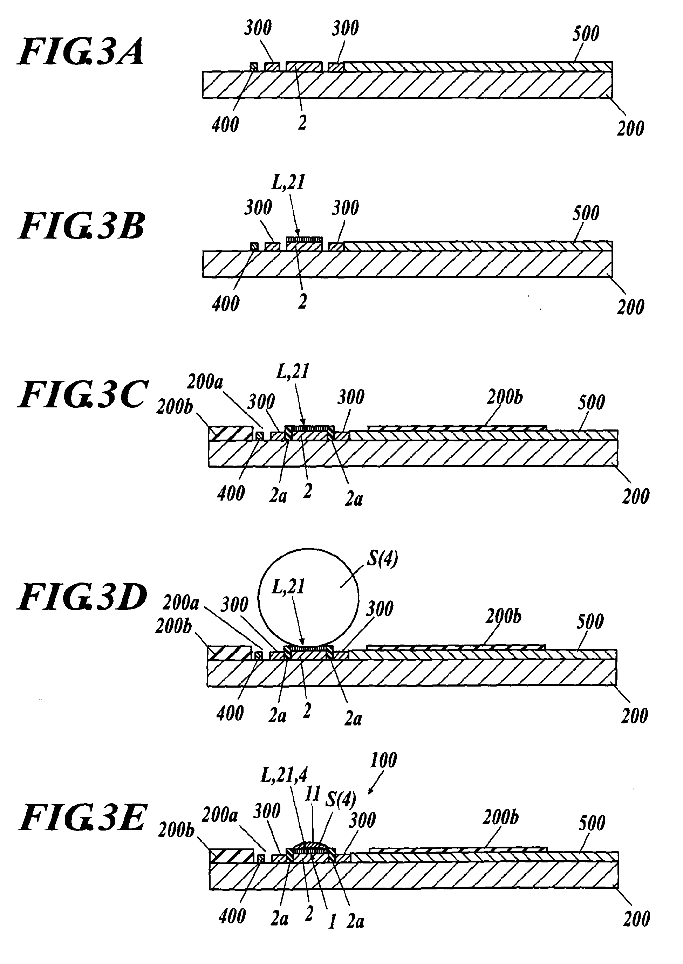

A manufacturing method of the enzyme sensor 100 is described with reference to FIGS. 3A-3E.

First, for example, a pattern of a three-pole structure of a working electrode (electrode 2), the counter electrode 300, and the reference electrode 400 is made on the substrate 200 as shown in FIG. 3A.

To put it concretely, the pattern of the three-pole structure of the working electrode (electrode 2), the counter electrode 300, and the reference electrode 400 is made on the substrate 200 by, for example, the publicly known photolithographic method, and the lift-off method or the etching method.

To put it more concretely, for example, a proper quantity of photoresist is applied to the substrate 200 by the use of a spin coater or the like. Next, the photoresist is exposed for several seconds by an ultraviolet exposing apparatus, and consequently the photomask pattern of the three-pole structure of the working electrode (electrode 2), the counter electrode 300, and the reference electrode 400 is transferred. Next, the post-bake processing of the photoresist is performed, following which the development of the photoresist is performed with a developing solution to form a pattern of the photoresist. Next, a metal thin film having a film thickness of, for example, about several hundreds nm is formed by a sputtering method, following which the resist is peeled off by the lift-off method to form a three-pole electrode.

Although the substrate 200 is not particularly limited as long as the substrate 200 is, for example, insulative here, it is desirable that the substrate 200 is a smooth substrate having a heat resisting property, which substrate is made of, for example, heat-resistant glass, silicon, quartz, or sapphire, in consideration of performing the synthesis of the carbon nanotubes 3.

Moreover, as the metal thin film formed by the sputtering method, for example, a precious metal, such as gold, platinum, or cupper, can be cited.

Incidentally, the method of making the pattern of the three-pole structure of the working electrode (electrode 2), the counter electrode 300, and the reference electrode 400 on the substrate 200 is not limited to the method mentioned above. For example, the forming method of the metal thin film is not limited to the sputtering method, but, for example, a vapor deposition method may be used.

Next, for example, the carbon nanotube layer L including the plurality of carbon nanotubes 3 is formed on the working electrode (electrode 2) as shown in FIG. 3B.

The carbon nanotubes 3 are directly synthesized on the electrode 2 and/or the metallic catalyst formed on the electrode 2, here. Hereby, the enzyme electrode 1 is led to have the characteristics of having no Schottky barriers between the electrode 2 and the carbon nanotubes 3, and of having small contact resistances.

Moreover, the intervals of the carbon nanotubes 3 are set to be the magnitudes such that the enzymes 4 can be put between the carbon nanotubes 3. That is, the intervals between the carbon nanotubes 3 are controlled according to the sizes (diameters) of the enzymes 4. To put it concretely, the density of the carbon nanotubes 3 is controlled by desired intervals (the intervals according to the sizes (diameters) of the enzymes 4 to be immobilized therein) within a range, for example, from 1 nm to 100 nm. When the enzymes 4 form multimeric complexes, then the sizes (diameters) of the enzymes 4 to be immobilized can be the sizes (diameters) of the multimeric complexes. The multimeric complexes are compounds produced by the bonding of two or more enzymes (proteins) with one another directly or with low-molecular substances, such as water, put between them. The bonding includes covalent bonding, ionic bonding, hydrogen bonding, and coordination bonding. However, the kinds of the bonding are not particularly limited.

Incidentally, the carbon nanotube layer L may be a single layer, multilayers, or the one in which both of the single layer and the multilayers are mixed.

To put it concretely, a desired minute pattern is formed on the working electrode (electrode 2) by, for example, the photolithographic method, the nanoimprint method, or the like, and a metallic catalyst pattern is carried by the pattern by an impregnating method or the like. Next, the carbon nanotubes 3 are grown from the metallic catalyst pattern as a starting point by a chemical vapor deposition method (CVD method) or the like.

Here, as the metallic catalyst, for example, an active metal, such as iron, cobalt, or nickel, is desirable.

Incidentally, the method of forming the carbon nanotube layer L including the plurality of carbon nanotubes 3 on the working electrode (electrode 2) is not limited to the method described above. For example, the method of growing the carbon nanotubes 3 from the metallic catalyst is desirably a thermochemical vapor deposition method (TCVD method), if possible, in consideration of the temperature of the process and the like, but the method is not limited to the TCVD method.

Moreover, although the carbon nanotubes 3 are made to extend directly from the metallic catalyst immobilized on the working electrode (electrode 2), the extending method of the carbon nanotubes 3 is not limited to the one mentioned above. For example, the carbon nanotubes may be made to extend from the electrode 2 directly, or the carbon nanotubes 3 made to extend from the metallic catalyst directly and the carbon nanotubes 3 made to extend from the electrode 2 directly may be mixed.

As the method of making the carbon nanotubes 3 extend from the electrode 2 directly, for example, it is conceivable to form the working electrode (electrode 2) from a metal that functions as a metallic catalyst.

To put it concretely, for example, iron group metals, such as iron, cobalt, and nickel, alloys including at least one kind of the iron group metals, or metal oxides produced by oxidizing these iron group metals or the alloys by performing strong oxidation preferably by heat are used as the substrate 200, and the regions on the substrate 200 other than the working electrode (electrode 2), the counter electrode 300, and the reference electrode 400 are coated by an insulation film made of silicon, glass, or the like, by the sputtering method or the like. Thereby, the substrate 200 on which the three-pole electrode is formed is made. Then, the carbon nanotubes 3 are made to extend from the electrode 2 directly by growing the carbon nanotubes 3 from the working electrode (electrode 2) formed from a metal (iron group metal, the alloy thereof, or a metal oxide produced by oxidizing the iron group metal or the alloy) functioning as the metallic catalyst as the starting point by the chemical vapor deposition method (CVD method) or the like.

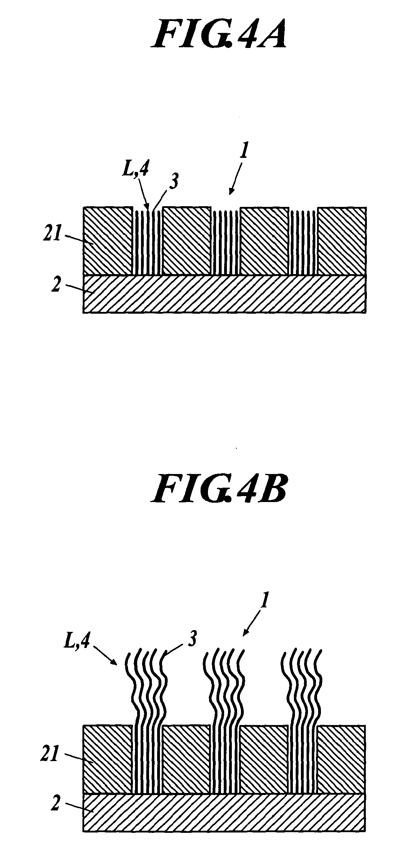

Moreover, although only the carbon nanotubes 3 are produced on the working electrode (electrode 2), the present invention is not limited to this case. For example, as shown in FIGS. 4A and 4B, an anodized film 21 including small cavities may be formed on the working electrode (electrode 2) by the anodization of aluminum, silicon, or the like, and the carbon nanotubes 3 may be produced in the small cavities on the working electrode (electrode 2).

To put it concretely, the anodized film 21 including the small cavities on the working electrode (electrode 2) is produced by, for example, the anodization of aluminum, silicon, or the like, and the metallic catalysts are embedded in the small cavities to grow the carbon nanotubes 3 from the metallic catalysts as starting points. The carbon nanotubes 3 may be thus formed in the small cavities on the working electrode (electrode 2).

Moreover, for example, a metallic catalyst layer made of a metallic catalyst is formed on a working electrode (electrode 2) made of a metal thin film of platinum or the like. A film made of aluminum, silicon, or the like, is formed on the metallic catalyst layer. Next, small cavities penetrating the film from the surface thereof to the surface of the metallic catalyst layer are formed by the anodization of the film, and consequently the anodized film 21 including the small cavities is produced on the working electrode (electrode 2). Then, the carbon nanotubes 3 may be formed in the small cavities on the working electrode (electrode 2) by growing the carbon nanotubes from the metallic catalyst layer exposed by the small cavities as the starting point.

Moreover, for example, the anodized film 21 including the small cavities penetrating the anodized film 21 from the surface thereof to the surface of the metallic catalyst layer is produced on the working electrode (electrode 2), and the metallic catalyst layer made of the metallic catalyst is formed on the anodized film 21. Then, for example, the substrate 200 is heated to diffuse the metallic catalyst. Thereby, the metallic catalyst is disappeared from the surface of the anodized film 21, so that metallic catalyst particles cohere in islands only in the small cavities on the working electrode (electrode 2). Then, the carbon nanotubes 3 are grown from the metallic catalyst particles as starting points, and the carbon nanotubes 3 may be thus formed in the small cavities on the working electrode (electrode 2).

Incidentally, the carbon nanotubes 3 formed in the small cavities of the anodized film 21 on the working electrode (electrode 2) are upward grown from the bottoms of the small cavities of the anodized film 21 as shown in FIGS. 4A and 4B. Then, it is conceivable that the carbon nanotubes 3 are formed in two different kinds of shapes according to the time of growing. That is, when the growing of the carbon nanotubes 3 are stopped at an insufficient growth stage, then, for example, as shown in FIG. 4A, the carbon nanotubes 3 formed in the small cavities of the anodized film 21 in parallel with the small cavities to have a good orientation can be obtained. On the other hand, by performing sufficient growth, the carbon nanotubes 3 that are formed in parallel with the small cavities of the anodized film 21 in the small cavities of the anodized film 21 and are randomly formed on the outside of the small cavities of the anodized film 21 can be obtained, as shown in FIG. 4B, for example.

Moreover, it is also possible to control the lengths of the carbon nanotubes 3 disposed in the small cavities of the anodized film 21 by the thickness of the anodized film 21.

Here, the enzymes 4 are sometimes put between the carbon nanotubes 3, or are sometimes in the state in which the enzymes 4 put between the carbon nanotubes 3 and the enzymes 4 twined around the carbon nanotubes 3 are mixed.

Moreover, the carbon nanotubes 3 on the outside of the small cavities of the anodized film 21 are sometimes intertwined with one another and are sometimes not intertwined with one another as shown in FIG. 4B, for example, and further are sometimes in the state in which the carbon nanotubes 3 intertwined with one another and the carbon nanotubes 3 not intertwined with one another are mixed.

Next, hydrophobic thin films, each made of, for example, SiO, are formed around the working electrode (electrode 2) and around the analysis section 200a by the sputtering method or the like, as shown in FIG. 3C, for example. In the following, the hydrophobic thin film formed around the working electrode (electrode 2) will be referred to as a hydrophobic insulating section 2a, and the hydrophobic thin film formed around the analysis section 200a will be referred to as the hydrophobic insulation film 200b.

Furthermore, the reference electrode 400, which is a silver/silver chloride electrode, is produced by applying, for example, silver/silver chloride ink onto the pattern of the reference electrode 400 in the produced pattern of the three-pole structure of the working electrode (electrode 2), the counter electrode 300, and the reference electrode 400, and by baking the applied silver/silver chloride ink.

Next, the enzymes 4 are immobilized in the carbon nanotube layer L.

To put it concretely, for example, as shown in FIG. 3D, an enzyme solution S is dropped on the carbon nanotube layer L formed on the working electrode (electrode 2) or formed in the small cavities of the anodized film 21 produced on the working electrode (electrode 2) with a pipette, a dispenser, or the like. Hereby, the enzymes 4 are physically immobilized in the carbon nanotube layer L.

On this occasion, the enzyme solution S dropped with the pipette, the dispenser, or the like, evaporates, touching only the working electrode (electrode 2) while keeping the sphere thereof, owing to the influence of the hydrophobic insulating section 2a formed around the working electrode (electrode 2). Then, the enzymes 4 are concentrated into a high concentration in the carbon nanotube layer L on the working electrode (electrode 2). Hereby, it becomes possible to attain the immobilization of the high density enzymes 4 only in the carbon nanotube layer L on the working electrode (electrode 2).

Incidentally, it is preferable to perform the drying processing of the carbon nanotube layer L in which the enzymes 4 are immobilized after the immobilization.

Next, for example, as shown in FIG. 3E, it is desirable to form a predetermined layer (hereinafter referred to as an immobilization layer 11) to function as an outflow preventing section to prevent the outflows of the enzymes 4 immobilized in the carbon nanotube layer L so as to cover the carbon nanotube layer L.

Here, the immobilization layer 11 is not especially limited as long as the layer, for example, prevents the outflows of the enzymes 4 immobilized in the carbon nanotube layer L and transmits a target substance. To put it concretely, the immobilization layer 11 may be, for example, hydrophilic or hydrophobic, may be an inorganic substance or an organic substance, may be a porous material or a fibrous material, may be a polymeric gel, may be a photo-crosslinking resin, or may be the other publicly known immobilization layers.

Moreover, carboxyl groups (outflow preventing section) to form amide bonds by reacting with the amine groups included in the enzymes 4, which is proteins, may be introduced into the ends of the carbon nanotubes 3 to immobilize the enzymes 4 into the carbon nanotube layer L by chemical bonding. The outflows of the enzymes 4 immobilized in the carbon nanotube layer L may be thereby prevented.

Moreover, the outflows of the enzymes 4 immobilized in the carbon nanotube layer L may be prevented by using a publicly known enzyme immobilization method.

To put it concretely, for example, a predetermined cross-linking agent (outflow preventing section) may be introduced into the carbon nanotube layer L. That is, for example, conductive polymeric molecules may be introduced into the carbon nanotubes 3, and then the enzymes 4 may be immobilized by the cross-linkage of the conductive polymeric molecules. Alternatively, the enzymes 4 may be immobilized by the cross-linkage of glutaraldehyde or the like, or the enzymes 4 may be immobilized by the cross-linkage of a photo-crosslinking resin or the like.

For example, when the carbon nanotubes 3 are cross-linked together with the enzymes 4 by using the conductive polymeric molecules, such as polyaniline molecules, then a plurality of carbon nanotubes 3 becomes the state of a network structure through a plurality of cross-linked parts. Thereby, the electrode structures of the carbon nanotubes 3 can be physically more strengthened, and at the same time their specific surface areas can be increased.
Consequently, the further improvement of the sensitivity of the enzyme electrode and the improvement of the response speed thereof are led.

Here, in order to prevent the outflows of the enzymes 4 immobilized in the carbon nanotube layer L, the immobilization layer 11 may cover the carbon nanotube layer L; the carboxyl groups may be introduced to the ends of the carbon nanotubes 3; or predetermined cross-linking agents may be introduced into the carbon nanotube layer L. Moreover, the outflows of the enzymes 4 immobilized in the carbon nanotube layer L may be prevented by using arbitrary two or three sections of the immobilization layer 11 to cover the carbon nanotube layer L, the carboxyl groups introduced into the ends of the carbon nanotubes 3, and the predetermined cross-linking agents introduced into the carbon nanotube layer L at the same time.

Incidentally, the outflow preventing section is not limited to the section using the immobilization layer 11, the section using the carboxyl groups, and the section using the predetermined cross-linking agents, but the section is arbitrary as long as the section can prevent the overflows of the enzymes 4 immobilized in the carbon nanotube layer L.

Now, the enzymes 4 are proteins each having a molecular weight of about ten thousands to about two hundred thousands, and consequently it is sometimes difficult for the active centers of the enzyme molecules to perform fast electron transfers with the electrode 2 or the carbon nanotubes 3. Accordingly, it is preferable to introduce electron carriers to accelerate the deliveries of electrons between the enzymes 4 and the electrode 2 or the carbon nanotubes 3 into the carbon nanotube layer L. Moreover, also in the case where the rates of reactions are limited by dissolved oxygen concentrations and only the samples of low concentrations cannot be measured, it is effective to introduce electron carriers into the carbon nanotube layer L with the object of the extension of the range of detection.

To put it concretely, as the electron carriers, for example, potassium ferricyanide molecules, ferrocene molecules, ferrocene derivative molecules, benzoquinone molecules, quinone derivative molecules, osmium complex molecules, and the like, are used.

Moreover, it is also preferable to introduce, for example, coenzymes to catalyze the expression of the activity of the enzymes 4 into the carbon nanotube layer L.

For example, when the reactions of the enzymes 4 with a target substance are the ones that do not easily proceed by the catalysis of the amino acid side chains of the enzymes 4, such as the reactions via instable intermediates, then the coenzymes that are low molecular weight organic compounds that have appropriate structures and participate in the expression of enzyme action are frequently used. In particular, when coenzyme-dependent enzymes are used as the enzymes 4, the enzyme action can be efficiently performed by introducing the coenzymes into the carbon nanotube layer L.

The coenzymes can be suitably selected according to the kinds of the enzymes 4 (coenzyme-dependent enzymes). To put it concretely, as the coenzymes, for example, one kind or the combination of two or more kinds of nicotinamide adenine dinucleotide (NAD⁺), nicotinamide adenine dinucleotide phosphate (NADP⁺), coenzyme I, coenzyme II, flavin mononucleotide (FMN), flavin adenine dinucleotide (FAD), lipoic acid, coenzyme Q, and the like, are cited. Among them, the coenzymes of NADs, such as the nicotinamide adenine dinucleotide (NAD⁺) and the nicotinamide adenine dinucleotide phosphate (NADP⁺), are used.

When the electron carriers or the coenzymes are introduced into the carbon nanotube layer L, the electron carriers and the coenzymes may be immobilized in the carbon nanotube layer L by the use of, for example, the cross-linking agents, such as the glutaraldehyde and a photo-crosslinking resin, or the electron carriers and the coenzymes may be immobilized together with the enzymes 4 by dissolving the electron carriers and the coenzymes into the enzyme solution S. Alternatively, the electron carriers and the coenzymes may be immobilized by physically or chemically bonding them with the carbon nanotubes 3 as the conductive polymeric molecules. Moreover, the electron carriers and the coenzymes may be dissolved and dispersed in an electrolyte, and the electron carriers and the coenzymes may be disposed by dropping the electrolyte in the analysis section 200a at the time of the use of the enzyme electrode 1.

Now, as the electrochemical measurement method by the enzyme sensor 100, for example, a publicly known measurement method to measure an oxidation current or a reduction current, such as the chronoamperometry method, the coulometric method, or the cyclic voltammetry method, can be used. As a measurement method, any of a disposable method, a batch method, a flow-injection method, and the like, can be used.

Preferably, the main body of a measuring instrument to which the enzyme sensor 100 using the enzyme electrode 1 is attached includes, for example, the function capable of transmitting data to a personal computer by wire communication or wireless communication, and can confirm measured values in real time. Moreover, it is desirable to configure the main body to be able to attach a plurality of kinds of enzyme sensors 100, and to include the function of measuring the detected results by the plurality of kinds of the enzyme sensors 100 at the same time to enable the intercomparison of data and the examination thereof.

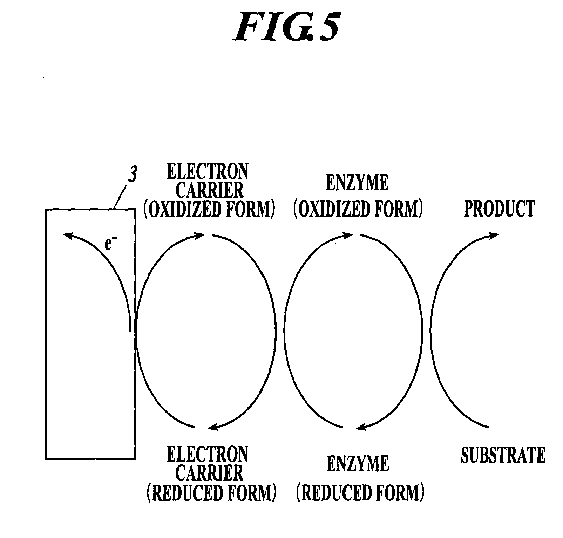

Next, with reference to FIG. 5, a description is given to the principle of measuring the concentration of a target substance in a sample by the electrochemical measurement method by the enzyme sensor 100 using the enzyme electrode 1 of the embodiment of the present invention.

In FIG. 5, for example, it is supposed that an oxidized form enzyme (enzyme 4) is immobilized in the carbon nanotube layer L by being put between carbon nanotubes 3. The immobilized oxidized form enzyme oxidizes the substrate, which is the target substance in the sample, by a selective catalysis, and becomes a reduced form enzyme. Next, when an voltage is applied between the working electrode (electrode 2) and the reference electrode 400 with the working electrode being plus, then the reduced form enzyme delivers an electron (e⁻) to the working electrode (electrode 2) or to the carbon nanotube electrode (carbon nanotube 3) formed on the working electrode (electrode 2) directly or indirectly through an electron carrier, and the reduced form enzyme restitutes to the oxidized form enzyme. On this occasion, a current to reoxidize the reduced form enzyme or the reduced form electron carrier flows between the working electrode (electrode 2) and the reference electrode 400. Because the current value is proportioned to the magnitude of the enzyme kinetics, that is, the concentration of the substrates included in the sample, it is possible to calculate the concentration of the target substance included in the sample on the basis of the current value.

To put it concretely, for example, when the concentration of glucose is measured as a target substance, then glucose oxidase enzymes and potassium ferricyanide molecules can be used as the enzymes 4 and the electron carriers, respectively. As shown in the following formula (1), glucose (C₆H₁₂O₆) is changed into gluconic acid (C₆H₁₂O₇) by an enzyme 4, and at the same time glucose gives electrons (e⁻) to ferricyanide ions ( [Fe (III) (CN)₆]³⁻), which are electron carriers, to reduce the ferricyanide ions to ferrocyanide ions ([Fe (II) (CN)₆]⁴⁻). The ferrocyanide ions reduced by the enzyme 4 are further oxidized to ferricyanide ions by the electrode 2 or the carbon nanotubes 3 as shown in the following formula (2). On the other hand, hydrogen ions (H⁺) receives electrons to produce water (H₂O) together with oxygen (O₂) at the counter electrode 300 as shown in the following formula (3). The concentration of glucose can be indirectly measured by measuring the current value at this time.

C₆H₁₂O₆+ 2[Fe(CN)₆]³⁻ + H₂O → C₆H₁₂O₇ + 2[Fe(CN)₆]⁴⁻ + 2H⁺ (1)

2[Fe(CN)₆]⁴⁻ → 2[Fe(CN)₆]³⁻ + 2e⁻ (2)

2H⁺ + 1/2O₂ + 2e⁻ → H₂O (3)

Incidentally, it is possible to avoid the influences of measurement disturbing materials and to measure a target substance selectively at high sensitivity by setting the voltage of the working electrode (electrode 2) to the reference electrode 400 at a specific voltage at the time of the measurement. The set voltage differs according to the target substance.

Moreover, it is necessary for the enzyme sensor 100 using the enzyme electrode 1 of the embodiment of the present invention to change the kinds of the enzymes 4 according to a target substance. To put it concretely, for example, when the target substance is glucose, ethanol, formaldehyde, and total cholesterol, then glucose oxidase or glucose dehydrogenase, alcohol oxidase or alcohol dehydrogenase, formaldehyde oxidase or formaldehyde dehydrogenase, and a mixture of cholesterol esterase and cholesterol oxidase, can be used, respectively, as the enzymes 4.

In the following, the embodiment of the present invention will be described by means of concrete examples.

### [First Example]

In a first example, a basic substrate was produced, and the enzyme electrode 1 was formed by immobilizing the enzymes 4 on the basic substrate to produce the enzyme sensor 100. Then, the enzyme sensor 100 was evaluated.

### (1) Production of Basic Substrate

First, the basic substrate was produced.

### (1-1) Production of Electrode

A pattern of the three-pole structure of the working electrode (electrode 2), the counter electrode 300, and the reference electrode 400 was produced on the substrate 200.

To put it concretely, for example, a substrate 200 made of silica glass was prebaked at 95°C for 90 seconds by the use of a hot plate. After that, 50 µL of a negative type resist was applied by the use of a spin coater, and a photomask pattern of the three-pole structure of the working electrode (electrode 2), the counter electrode 300, and the reference electrode 400 was transferred by the use of an ultraviolet exposing apparatus. Next, the substrate 200 was post-baked at 120°C for 60 seconds. After that, the substrate 200 was developed by a developing solution for 70 seconds, and was washed by means of distilled water. Next, a metal thin film (platinum thin film) having a film thickness of 800 nm was formed by the sputtering method. After that, the substrate 200 was soaked in acetone while being washed therein by an ultrasonic wave by the lift-off method for 30 minutes, and thereby the resist was peeled off. Thus a platinum electrode was formed. The film formation conditions of the platinum layer were set as follows: the degree of vacuum was 10⁻⁵ Pa; the substrate temperature was 60°C; and the flow rate of the argon gas was 40 sccm.

### (1-2) Production of Anodized Film

Next, the anodized film 21 of a porous body including small cavities was formed on the working electrode (electrode 2).

To put it concretely, for example, a Ti layer having a film thickness of 100 nm was formed on the working electrode (electrode 2) as an undercoat, and an Al layer having a film thickness of 500 nm was formed on the Ti layer, by the sputtering method. Next, the working electrode was soaked into oxalic acid aqueous solution (0.3 M) at 17°C, and the anodization processing of the working electrode (electrode 2) was performed by applying a DC voltage of 40 V to the working electrode. The anodization current of the working electrode was monitored during the anodization processing in order to detect the progress of the anodization up to the Ti film. Penetrated nanoholes (small cavities) were formed at the same time as Al being oxidized to be the alumina of an insulating layer by the anodization processing. Then, after the anodization processing, the working electrode was washed by the distilled water and isopropyl alcohol. When the surface of the anodized film 21 produced on the working electrode (electrode 2) was observed with a transmission electron microscope (TEM), it was confirmed that small cavities, each having a diameter of about 60 nm, were formed with intervals of about 300 nm.

### (1-3) Production of Carbon Nanotubes

Next, the carbon nanotubes 3 were produced on the working electrode (electrode 2).

To put it concretely, for example, the substrate 200 was soaked in cobalt nitrate aqueous solution (0.2 M) for 10 minutes. After the substrate 200 was pulled up, the substrate 200 was heated at 400°C for three hours in the air to carry cobalt particles on the surface of the working electrode (electrode 2) in the small cavities of the anodized film 21 to form a metallic catalyst pattern. After that, thermochemical vapor deposition reactions (TCVD method) were caused by means of a thermochemical vapor phase growth furnace to form the carbon nanotubes 3 on the working electrode (electrode 2) directly with cobalt as a catalyst. The supplied gases were an argon gas of a flow rate of 360 sccm and propylene of a flow rate of 120 sccm as a carbon source. The reaction temperature was set to be 700°C; the reaction time was set to be for eight minutes; the pressure was set to be 0.1 MPa. The carbon nanotubes formed on the working electrode (electrode 2) directly by the reaction had a diameter of about 10 nm each, and were formed in a pattern with intervals of about eight nm.

### (1-4) Finish of Basic Substrate

Next, the finish of the basic substrate was performed.

To put it concretely, a SiO thin film having a film thickness of 500 nm was formed around the working electrode (electrode 2) and the analysis section 200a by the sputtering method, and thereby the hydrophobic insulating section 2a and the hydrophobic insulation film 200b were produced around the working electrode (electrode 2) and the analysis section 200a, respectively. Next, a silver/silver chloride ink (available from BAS Inc.) was applied on the pattern of the reference electrode 400, and the reference electrode 400 was baked at 120°C to produce the reference electrode 400, which was a silver/silver chloride electrode.

The basic substrate was produced as mentioned above.

### (2) Production of Enzyme Sensor 100

Next, by immobilizing the enzymes 4 in the carbon nanotube layer L included in the basic substrate, the enzyme electrode 1 was formed, and the enzyme sensor 100 was produced.

As the enzymes 4, formaldehyde dehydrogenase, which was a coenzyme (NAD⁺) dependent type enzyme, was used.

To put it concretely, for example, first, 10 µmol of naphthaquinone was dissolved in 1000 µL of a phosphate buffer (pH 7.5) to produce a solution A. Moreover, 0.25 µmol of NAD⁺ and 0.5 U of formaldehyde dehydrogenase were mixed in 100 µL of the phosphate buffer (pH 7.5), and the mixed solution was agitated and dissolved at 4°C for 30 minutes to produce a solution B. Furthermore, 50 mL of a photo-crosslinking resin (available from Toyo Gosei Co., Ltd.) was dissolved in 50 mL of the phosphate buffer, and the pH of the solution was adjusted to 7.5 to produce a solution C.

Then, 20 µL of the solution A was extracted with a micropipette and was dropped on the working electrode (carbon nanotube layer L) to let the solution A dry naturally at a room temperature (25°C) for three hours. Next, 20 µL of the solution B was extracted with the micropipette, and was dropped on the working electrode (carbon nanotube layer L) to let the solution B dry naturally at the room temperature (25°C) for three hours. The dropped solutions touched only the working electrode (carbon nanotube layer L) by the hydrophobic insulating section 2a around the working electrode (electrode 2), and evaporated, keeping their shape in a sphere each. Furthermore, 10 µL of the solution C was extracted with the micropipette, and was dropped on the working electrode (carbon nanotube layer L) to be photocrosslinked by the irradiation of an ultraviolet ray having a wavelength of 360 nm. Then, the solution C was left at rest at the room temperature (25°C) for two hours. In such a way, the enzyme electrode 1 was formed, and the enzyme sensor 100 was obtained.

Incidentally, in place of extracting 10 µL of the solution C with the micropipette to drop the extracted solution C onto the working electrode (carbon nanotube layer L), 1 µL of 2%(v/v) glutaraldehyde solution may be extracted with the micropipette to be applied onto the working electrode (carbon nanotube layer L).

### (3) Evaluation of Enzyme Sensor 100

Next, control experiments were performed to evaluate the enzyme sensor 100.

First, as the control experiments, a conventional enzyme sensor [1] and a conventional enzyme sensor [2] were produced. The enzyme sensor [1] immobilized enzymes by mixing carbon nanotubes and the enzymes (formaldehyde dehydrogenase) with a mineral oil to apply the mixed solution to a platinum electrode (working electrode). The enzyme sensor [2] immobilized enzymes by applying only the enzymes (formaldehyde dehydrogenase) to a platinum electrode (working electrode).

To put it concretely, in order to produce the conventional enzyme sensor [1], for example, 10 mg of carbon nanotubes, 1 µmol of naphthaquinone, 0.25 µmol of NAD⁺, and 0.5 U of formaldehyde dehydrogenase were mixed in 100 µL of a phosphate buffer (pH 7.5), in which 50 µL of mineral oil was introduced, and the mixed solution was agitated and dissolved at 4°C for 30 minutes. 20 µL of the solution was extracted with a micropipette, and was dropped on the working electrode (electrode 2) produced as described in "(1-1) Production of Electrode" to concentrate the solution and to immobilize the enzymes on the working electrode. Furthermore, 10 µL of the solution C was extracted with the micropipette, and was dropped on the working electrode. Then, the photocrosslinking of the solution C was performed by the irradiation of an ultraviolet ray having a wavelength of 360 nm. The conventional enzyme sensor [1] was obtained by such a way.

Moreover, in order to produce the conventional enzyme sensor [2], for example, 1 µmol of naphthaquinone, 0.25 µmol of NAD⁺, and 0.5 U of formaldehyde dehydrogenase were mixed in 100 µL of a phosphate buffer (pH 7.5), and the mixed solution was agitated and dissolved at 4°C for 30 minutes. 20 µL of the solution was extracted with a micropipette, and was dropped on the working electrode (electrode 2) produced as described in "(1-1) Production of Electrode" to concentrate the solution and to immobilize the enzymes on the working electrode. Furthermore, 10 µL of the solution C was extracted with the micropipette, and was dropped on the working electrode. Then, the photocrosslinking of the solution C was performed by the irradiation of an ultraviolet ray having a wavelength of 360 nm. The conventional enzyme sensor [2] was obtained by such a way.

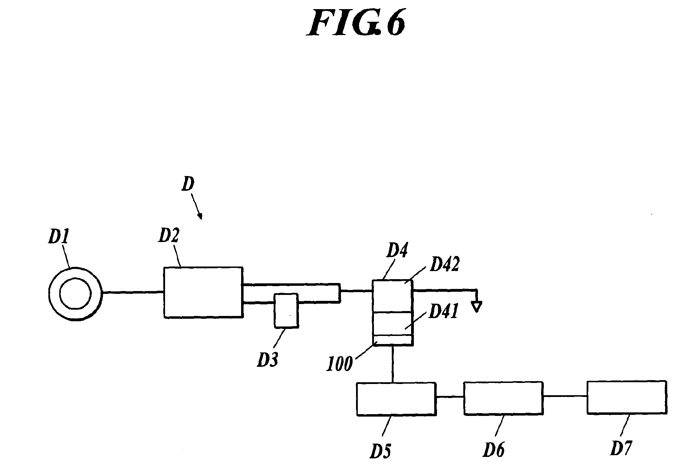

Next, a measuring device D to evaluate the enzyme sensor 100 of the embodiment of the present invention, the conventional enzyme sensor [1], and the conventional enzyme sensor [2] is described with reference to FIG. 6.

The measuring device D is composed of, for example, a standard air generator D1, a gas generator D2, a vapor bubbler D3, a micro chamber D4, a potentiostat D5, an A/D converter D6, a computer D7 and the like.

The micro chamber D4 includes a microcell D41 for a liquid phase and a microcell D42 for a gaseous phase with a hydrophobic porous film put between them.

The size of the microcell D41 for the liquid phase coincides with the size of the opening portion formed in the top surface of the analysis section 200a, and the enzyme sensor 100 (enzyme sensor 100, conventional enzyme sensor [1], and conventional enzyme sensor [2]) is set so that the upper part of the analysis section 200a may be disposed on the lower side of the microcell D41 for the liquid phase with an O ring put between the upper part and the lower side.

The microcell D42 for the gaseous phase is configured so that a normal concentration formaldehyde gas may be introduced from the gas generator D2.

Hereby, the formaldehyde, which is the substrate (target substance) of the enzymes 4 (formaldehyde dehydrogenase), is supplied from the microcell D42 for the gaseous phase into the analysis section 200a through the hydrophobic porous film and the microcell D41 for the liquid phase.

The working electrode (electrode 2), the counter electrode 300, and the reference electrode 400 are connected to the potentiostat D5 (BAS-100B available from BAS Inc.) from the corresponding pads 5.00, respectively, through lead wires.

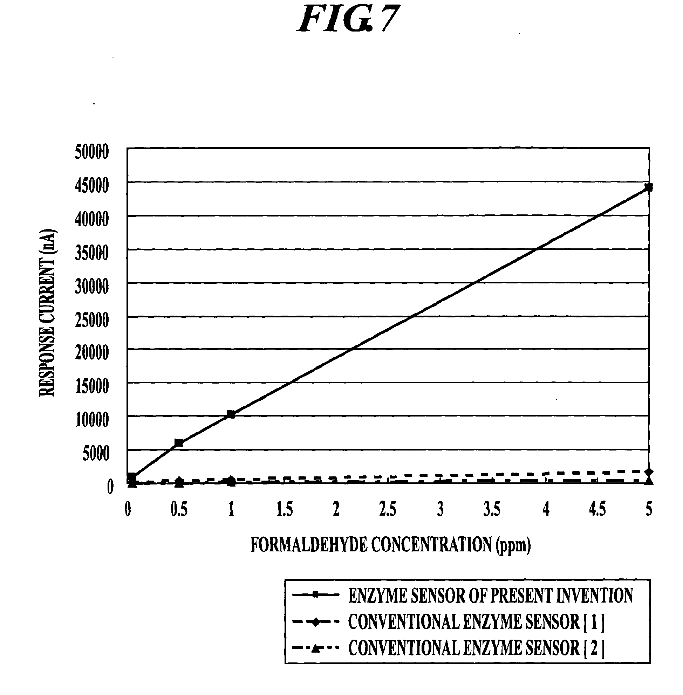

30 µL of the phosphate buffer (0.1 mM, pH 7.5) was dropped onto the microcell D41 for the liquid phase as an electrolyte in the measuring device D, and the electric potential of +100 mV was applied to the reference electrode 400 as against the working electrode. Then, a formaldehyde gas the concentration of which was continuously changed by the gas generator D2 at the room temperature (25°C) was introduced into the microcell D42 for the gaseous phase, and currents were measured by the current measurement by the amperometry method. The results are shown in FIGS. 7-9.

In FIG. 7, the abscissa axis indicates formaldehyde concentrations; the ordinate axis indicates response currents; a solid line and plotted quadrilaterals (■) indicate the data of the enzyme sensor 100 of the present invention; a broken line and plotted rhombuses (◆) indicate the data of the conventional enzyme sensor [1]; and an alternate long and short dash line and plotted triangles (A) indicate the data of the conventional enzyme sensor [2].

According to FIG. 7, the following was found. That is, when the formaldehyde concentration was 0.5 ppm, then the enzyme sensor 100 of the embodiment of the present invention had the 15-fold detection sensitivity as compared with that of the conventional enzyme sensor [1], and 117-fold detection sensitivity as compared with that of the conventional enzyme sensor [2]. That is, it was found that the enzyme sensor 100 of the embodiment of the present invention was considerably improved in the detection sensitivity thereof as compared with those of the conventional enzyme sensor [1] and the conventional enzyme sensor [2]. Moreover, it was found that a linear response region in a high concentration region was also notably improved. Hereby, it was found that the enzyme sensor 100 of the embodiment of the present invention was able to detect a target substance at high sensitivity.

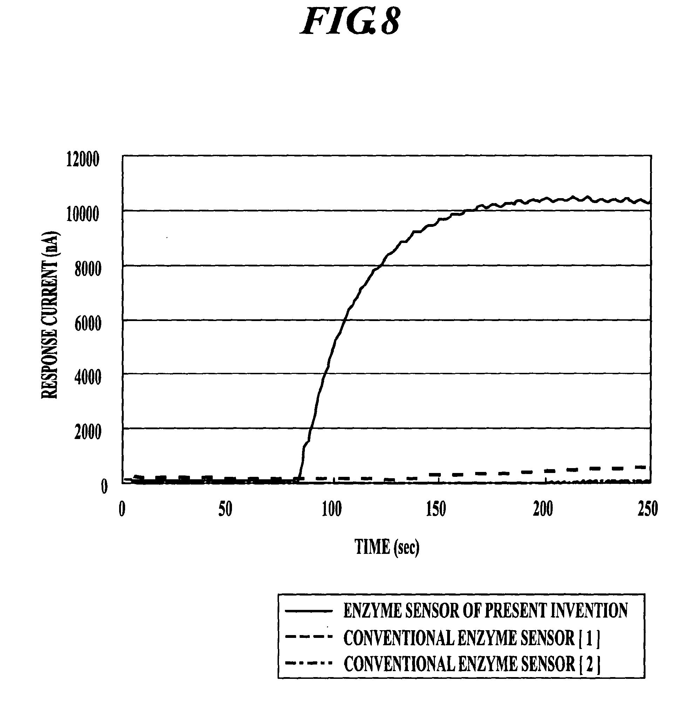

In FIG. 8, the abscissa axis indicates time, and the ordinate axis indicates response currents. FIG. 8 shows the changes of the output response currents when 1 ppm formaldehyde was introduced at the time point of 50 seconds into the enzyme sensor 100 of the embodiment of the present invention shown by the solid line, the conventional enzyme sensor [1] shown by the broken line, and the conventional enzyme sensor [2] shown by the alternate long and short dash line.

According to FIG. 8, it was found that the enzyme sensor 100 of the embodiment of the present invention showed a response to the introduction of formaldehyde after 30 seconds of the introduction as against 100 seconds of the conventional enzyme sensor [1] and 150 seconds of the conventional enzyme sensor [2]. That is, it was found that the response time of the enzyme sensor 100 of the embodiment of the present invention was considerably short as compared with those of the conventional enzyme sensor [1] and the conventional enzyme sensor [2]. Moreover, it was found that the inclination of an output response current of the enzyme sensor 100 of the embodiment of the present invention was also large as compared with those of the conventional enzyme sensor [1] and the conventional enzyme sensor [2]. Hereby, it was found that the enzyme sensor 100 of the embodiment of the present invention was able to detect a target substance at a high speed.

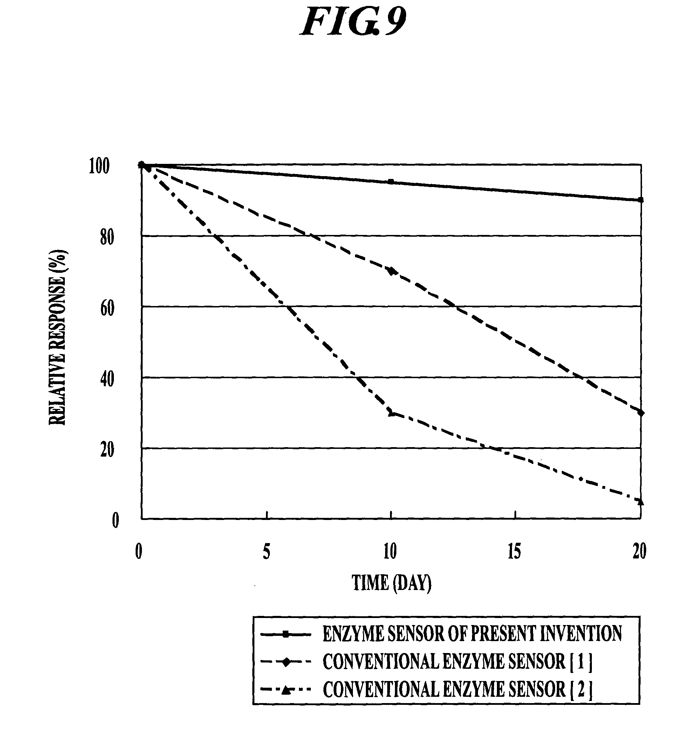

In FIG. 9, the abscissa axis indicates time, and the ordinate axis indicates relative responses (that is, response currents in the case where the response current on the first day was set to 100%). FIG. 9 shows the relative responses of the enzyme sensor 100 of the embodiment of the present invention shown by the solid line and the plotted quadrilaterals (■), the conventional enzyme sensor [1] shown by the broken line and the plotted rhombuses (◆), and the conventional enzyme sensor [2] shown by the alternate long and short dash line and the plotted triangles (A).

According to FIG. 9, the aged deterioration of the response current of the enzyme sensor 100 of the embodiment of the present invention was very small as compared with those of the conventional enzyme sensor [1] and the conventional enzyme sensor [2], and the relative response of the enzyme sensor 100 was 90% even after 20 days. Hereby, it was found that the enzyme sensor 100 of the embodiment of the present invention had an excellent stability and a longer operating life.

According to the enzyme electrode 1 of the embodiment of the present invention and the enzyme sensor 100 using the enzyme electrode 1, which have been described above, the enzyme electrode 1 is equipped with the electrode 2, the carbon nanotube layer L including the plurality of carbon nanotubes 3 extending from the electrode 2 and/or the metallic catalyst immobilized on the electrode 2 directly, and the enzymes 4 put between the carbon nanotubes 3 to be thereby immobilized in the carbon nanotube layer L.

That is, because the enzymes 4 can be securely immobilized in the carbon nanotube layer L by putting the enzymes 4 between the carbon nanotubes 3, the changes of the steric structures of the enzymes 4 are prevented, and consequently it is possible to provide the enzyme electrode 1 having excellent stability and a longer operating life, and the enzyme sensor 100 using the enzyme electrode 1 (see, for example, the results of FIG. 9).

Moreover, because the carbon nanotube layer L has a very large specific surface area, the carbon nanotube layer L can immobilize the enzymes 4 at a large amount of adsorption and to be high concentration. Because the carbon nanotubes 3 extend from the electrode 2 and/or the metallic catalyst immobilized on the electrode 2 directly, the enzyme electrode 1 has a characteristic of forming no Schottky barriers between the carbon nanotubes 3 and the electrode 2, and consequently it is possible to provide the enzyme electrode 1 capable of detecting a target substance at high sensitivity and the enzyme sensor 100 using the enzyme electrode 1 (see, for example, the results in FIG. 7).

Moreover, for example, from the results of FIG. 7, it was found that the enzyme sensor 100 of the embodiment of the present invention was able to detect a target substance at very high sensitivity even in the a low concentration region or in a high concentration region, that is, that the enzyme sensor 100 was a sensor having wide detection region.

Furthermore, the carbon nanotubes 3 also assume the role of the electrode 2, and the fact means that the enzymes 4 are put between carbon nanotube electrodes (carbon nanotubes 3). Consequently, the deliveries of electrons between the enzymes 4 and the carbon nanotube electrodes (carbon nanotubes 3) can be efficiently performed, and it is possible to provide the enzyme electrode 1 capable of detecting a target substance at a high speed and the enzyme sensor 100 using the enzyme electrode 1 (see, for example, the results of FIG. 8).

Moreover, for example, when quinone is used as electron carriers and a gold electrode is used as the electrode 2, then the enzyme electrode 1 and the enzyme sensor 100 using the enzyme electrode 1 have further advantages.

To put it concretely, the quinone system electron carriers are not reduced by the gold electrode at all, and do not function as the electron carriers. However, when quinone is introduced into the carbon nanotube layer L formed on the gold electrode (electrode 2) as the electron carriers, then the quinone is rapidly reduced to hydroquinone. That is, the quinone is led to work as excellent electron carriers.

Consequently, when the enzyme electrode 1 of the embodiment of the present invention and the enzyme sensor 100 using the enzyme electrode 1 are used, then the quinone system electron carriers can perform the sufficient function even when the electrode 2 is the gold electrode.

Incidentally, the present invention is not limited to the embodiment described above, and can be suitably changed without departing from the spirit and the scope of the invention.

For example, although the anodized film 21 was formed on the electrode 2 to form the carbon nanotubes 3 in the small cavities of the anodized film 21 on the electrode 2 in the first example, it in not always necessary to form the anodized film 21 on the electrode 2.

A concrete production method of a basic substrate in this case is illustrated in the following.

### (A) Production of Electrode

First, a pattern of the three-pole structure of the working electrode (electrode 2), the counter electrode 300, and the reference electrode 400 was produced on the substrate 200.

To put it concretely, for example, the substrate 200 made of silica glass was prebaked at 95°C for 90 seconds with a hot plate. After that, 50 µL of a negative type resist was applied on the substrate 200 with a spin coater, and a photomask pattern of the three-pole structure of the working electrode (electrode 2), the counter electrode 300, and the reference electrode 400 was transferred with an ultraviolet exposing apparatus. Next, the substrate 200 was post-baked at 120°C for 60 seconds, following which the substrate 200 was developed for 70 seconds in a developing solution and was washed by distilled water. Next, a metal thin film (platinum thin film) was formed on the substrate 200 to be a film thickness of 800 nm by the sputtering method. After that, the substrate 200 was soaked in acetone to be washed by an ultrasonic wave for 30 minutes, and thereby the resist was peeled off to form a platinum electrode by the lift-off method. The film formation conditions of the platinum layer were set as follows: the degree of vacuum was 10⁻⁵ Pa; the substrate temperature was 60°C; the flow rate of an argon gas was 40 sccm.

### (B) Production of Carbon Nanotubes

Next, the carbon nanotubes 3 were produced on the working electrode (electrode 2).

To put it concretely, 50 µL of a negative type resist was applied on a working electrode (electrode 2) by a spin coater, and a photomask pattern having a shape of a metallic catalyst pattern (for example, the sizes of the pattern were as follows: each of the opening diameters thereof was 2 µm, each of the opening intervals thereof was 4 µm) was transferred onto the working electrode (electrode 2) by an ultraviolet exposing apparatus. Next, the substrate 200 was post-baked at 120°C for 60 seconds. After that, the substrate 200 was developed by a developing solution for 70 seconds, and the substrate 200 was washed by distilled water.

Next, a nickel thin film having a film thickness of 100 nm was formed by means of a vacuum evaporator. After that, the substrate 200 was soaked in acetone and was washed by an ultrasonic wave for 30 minutes by the lift-off method. Thereby, the resist was peeled off, and a metallic catalyst pattern of the nickel thin film was formed on the working electrode (electrode 2). The film formation conditions were set as follows: the degree of vacuum was 10⁻⁵ Pa; the substrate temperature was 60°C; and the flow rate of an argon gas was 40 sccm.

Next, a thermochemical vapor deposition reaction (TCVD method) was performed by means of a thermochemical vapor phase growth furnace, and thereby the carbon nanotubes 3 were directly formed on the working electrode (electrode 2) with nickel as metallic catalysts.

### (C) Finish of Basic Substrate

Next, the finish of the basic substrate was performed.

To put it concretely, by forming a SiO thin film having a film thickness of 500 nm around the working electrode (electrode 2) and the analysis section 200a by the sputtering method, the hydrophobic insulating section 2a was produced around the working electrode (electrode 2), and the hydrophobic insulation film 200b was produced around the analysis section 200a. Next, a silver/silver chloride ink (available from BAS Inc.) was applied onto the pattern of the reference electrode 400, and the reference electrode 400 was baked at 120°C to produce the reference electrode 400 that was a silver/silver chloride electrode.

As mentioned above, the basic substrate in which only the carbon nanotube layer L was formed on the electrode 2 was produced. By immobilizing the enzymes 4 into the carbon nanotube layer L included in the basic substrate, the enzyme sensor 100 was produced.

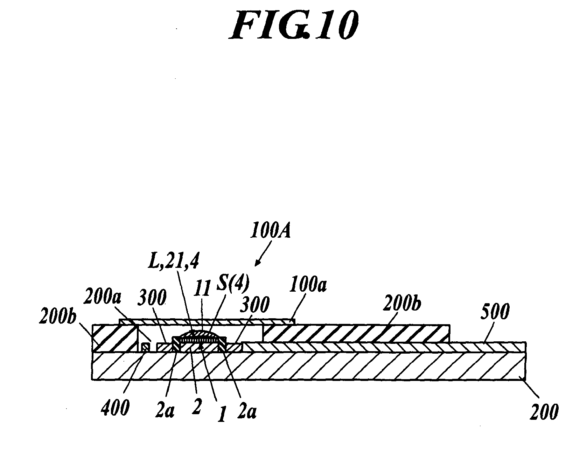

Moreover, the enzyme sensor of the embodiment of the present invention may be provided with a gas transmitting film 100a as a predetermined film, as, for example, an enzyme sensor 100A shown in FIG. 10, for covering the opening portion of the analysis section 200a, suppressing the transmission of liquids, and transmitting gas molecules.

By the provision of the gas transmitting film 100a, it becomes possible to transmit only the gas molecules to the side of the enzyme electrode 1 (the transmission from the outside of the analysis section 200a to the inside of the analysis section 200a) while suppressing the transmission of the electrolyte accumulated in the analysis section 200a (the transmission from the inside of the analysis section 200a to the outside of the analysis section 200a), and to detect the gas molecules in the enzyme electrode 1.

Although the minute electrodes produced by a photolithographic technique, for example, as shown in FIG. 1 and the like, are used as the examples of the working electrode (electrode 2), the counter electrode 300, and the reference electrode 400, these electrodes are not limited to the shown magnitudes, shapes, and configurations particularly.

To put it concretely, for example, these electrodes may be large ones to be used for a commercially available electrolytic cell, a measurement cell, and the like, or may be a disk electrode, a rotation ring disk electrode, a fiber electrode, and the like. Furthermore, minute electrodes (disk electrode, cylinder electrode, belt electrode, arranged belt electrode, arranged disk electrode, ring electrode, spherical electrode, comb-like electrode, pair electrodes, and the like) made by, for example, a publicly known microprocessing technique such as photolithographic technique may be used.

According to a first aspect of the preferred embodiment of the present invention, there is provided an enzyme electrode comprising:
an electrode;
a carbon nanotube layer including a plurality of carbon nanotubes extending directly from the electrode and/or a metallic catalyst immobilized on the electrode; and
an enzyme immobilized in the carbon nanotube layer by being sandwiched between the carbon nanotubes.

Preferably, the enzyme electrode further comprises an outflow preventing section to prevent the enzyme immobilized in the carbon nanotube layer from outflowing.

Preferably, the outflow preventing section is a predetermined layer to cover the carbon nanotube layer.

Preferably, the outflow preventing section is a predetermined cross-linking agent introduced in the carbon nanotube layer.

Preferably, the outflow preventing section is a carboxyl group introduced in an end of the carbon nanotubes, the carboxyl group reacting with an amine group of the enzyme to form an amide bond.

Preferably, an electron carrier to accelerate delivery of electrons between the enzyme and the electrode or the carbon nanotubes, and/or a coenzyme to catalyze expression of activity of the enzyme, are introduced in the carbon nanotube layer.

Preferably, the enzyme electrode further comprises a hydrophobic insulating section provided around the electrode.

According to a second aspect of the preferred embodiments of the present invention, there is provided an enzyme electrode comprising:
an electrode;
a carbon nanotube layer including a plurality of carbon nanotubes extending directly from the electrode and/or a metallic catalyst immobilized on the electrode;
an enzyme immobilized in the carbon nanotube layer by being sandwiched between the carbon nanotubes;
an outflow preventing section to prevent the enzyme immobilized in the carbon nanotube layer from outflowing;
an electron carrier to accelerate delivery of electrons between the enzyme and the electrode or the carbon nanotubes, and/or a coenzyme to catalyze expression of activity of the enzyme, the electron carrier and/or the coenzyme being introduced in the carbon nanotube layer; and
a hydrophobic insulating section provided around the electrode.

Preferably, an enzyme sensor to detect a target substance by an electrochemical measurement method, comprises the enzyme electrode.

Preferably, the enzyme sensor further comprises:
a substrate; and
an analysis section provided on a top surface of the substrate, wherein
the enzyme electrode is disposed inside the analysis section on the top surface of the substrate.

Preferably, the enzyme sensor further comprises a hydrophobic insulation film provided around the analysis section on the top surface of the substrate.

Preferably, an upper surface of the analysis section includes an opening portion, and the enzyme sensor further comprises a predetermined film to cover the opening portion, to suppress transmission of a liquid, and to transmit a gas molecule.

Preferably, an enzyme sensor to detect a target substance by an electrochemical measurement method, comprises:
the enzyme electrode;
a substrate;
an analysis section provided on a top surface of the substrate, the analysis section including an opening portion on an upper surface of the analysis section;
a hydrophobic insulation film provided around the analysis section on the top surface of the substrate; and
a predetermined film to cover the opening portion, to suppress transmission of a liquid, and to transmit a gas molecule, wherein
the enzyme electrode is disposed inside the analysis section on the top surface of the substrate.

According to the preferred embodiment of the present invention, in an enzyme electrode and an enzyme sensor using the enzyme electrode, the enzyme electrode includes:
an electrode; a carbon nanotube layer including a plurality of carbon nanotubes extending from the electrode and/or a metallic catalyst immobilized on the electrode directly; and enzymes immobilized in the carbon nanotube layer by being put between the carbon nanotubes.

That is, because the enzymes can be securely immobilized in the carbon nanotube layer by putting the enzymes between the carbon nanotubes, the changes of the steric structures of the enzymes can be prevented, and it is possible to provide an enzyme electrode having excellent stability and a longer operating life, and an enzyme sensor using the enzyme electrode.

Moreover, because the carbon nanotube layer has a very large specific surface area, the carbon nanotube layer can immobilize the enzymes in a large amount of adsorption to a high concentration. Because the carbon nanotubes extend from the electrode and/or the metallic catalyst immobilized on the electrode directly, the enzyme electrode has a characteristic of forming no Schottky barriers between the carbon nanotubes and the electrode, and consequently it is possible to provide an enzyme electrode capable of detecting a target substance at high sensitivity and an enzyme sensor using the enzyme electrode.

Furthermore, the carbon nanotubes assume the roles of electrodes, and thereby the deliveries of electrons between the enzymes and the carbon nanotube electrodes (carbon nanotubes) can be effectively performed because the enzymes are put between the carbon nanotube electrodes (carbon nanotubes). Thus it is possible to provide an enzyme electrode capable of detecting a target substance at a high speed, and an enzyme sensor using the enzyme electrode.

The entire disclosure of Japanese Patent Application No. 2007-176200 filed on July 4, 2007 including description, claims, drawings, and abstract are incorporated herein by reference in its entirety.

Although various exemplary embodiments have been shown and described, the invention is not limited to the embodiments shown. Therefore, the scope of the invention is intended to be limited solely by the scope of the claims that follow.

## Claims

1. An enzyme electrode comprising:
an electrode;
a carbon nanotube layer including a plurality of carbon nanotubes extending directly from the electrode and/or a metallic catalyst immobilized on the electrode; and
an enzyme immobilized in the carbon nanotube layer by being sandwiched between the carbon nanotubes.

2. The enzyme electrode according to claim 1, further comprising an outflow preventing section to prevent the enzyme immobilized in the carbon nanotube layer from outflowing.

3. The enzyme electrode according to claim 2, wherein the outflow preventing section is a predetermined layer to cover the carbon nanotube layer.

4. The enzyme electrode according to claim 2 or 3, wherein the outflow preventing section is a predetermined cross-linking agent introduced in the carbon nanotube layer.

5. The enzyme electrode according to any one of the claims 2-4, wherein the outflow preventing section is a carboxyl group introduced in an end of the carbon nanotubes, the carboxyl group reacting with an amine group of the enzyme to form an amide bond.

6. The enzyme electrode according to any one of the claims 1-5, wherein an electron carrier to accelerate delivery of electrons between the enzyme and the electrode or the carbon nanotubes, and/or a coenzyme to catalyze expression of activity of the enzyme, are introduced in the carbon nanotube layer.

7. The enzyme electrode according to any one of the claims 1-6, further comprising a hydrophobic insulating section provided around the electrode.

8. An enzyme electrode comprising:
an electrode;
a carbon nanotube layer including a plurality of carbon nanotubes extending directly from the electrode and/or a metallic catalyst immobilized on the electrode;
an enzyme immobilized in the carbon nanotube layer by being sandwiched between the carbon nanotubes;
an outflow preventing section to prevent the enzyme immobilized in the carbon nanotube layer from outflowing;
an electron carrier to accelerate delivery of electrons between the enzyme and the electrode or the carbon nanotubes, and/or a coenzyme to catalyze expression of activity of the enzyme, the electron carrier and/or the coenzyme being introduced in the carbon nanotube layer; and
a hydrophobic insulating section provided around the electrode.

9. An enzyme sensor to detect a target substance by an electrochemical measurement method, comprising the enzyme electrode according to any one of the claims 1-8.

10. The enzyme sensor according to claim 9, further comprising:
a substrate; and
an analysis section provided on a top surface of the substrate, wherein
the enzyme electrode is disposed inside the analysis section on the top surface of the substrate.

11. The enzyme sensor according to claim 10, further comprising a hydrophobic insulation film provided around the analysis section on the top surface of the substrate.

12. The enzyme sensor according to claim 10 or 11, wherein an upper surface of the analysis section includes an opening portion, and
the enzyme sensor further comprises a predetermined film to cover the opening portion, to suppress transmission of a liquid, and to transmit a gas molecule.

13. An enzyme sensor to detect a target substance by an electrochemical measurement method, comprising:
the enzyme electrode according to any one of the claims 1-8;
a substrate;
an analysis section provided on a top surface of the substrate, the analysis section including an opening portion on an upper surface of the analysis section;
a hydrophobic insulation film provided around the analysis section on the top surface of the substrate; and
a predetermined film to cover the opening portion, to suppress transmission of a liquid, and to transmit a gas molecule, wherein
the enzyme electrode is disposed inside the analysis section on the top surface of the substrate.
